# EUROPEAN PATENT APPLICATION

(11) **EP 2 540 226 A1**
(43) Date of publication of application: **02.01.2013**
(21) Application number: 11005192.7
(22) Date of filing: 27.06.2011
(51) Int. Cl.: A61B 5/22, A61B 5/11

(54) **Data acquisition device representing a virtual object**

(71) Applicant: ETH Zurich, 8092 Zürich (CH)
(72) Inventor: Lambercy, Olivier, 8050 Zürich (CH); Gassert, Roger, 8620 Wetzikon (CH); Fluet, Marie-Christine, 8008 Zürich (CH)

(57) **Abstract**

A data acquisition device (10) representing a virtual object and adapted to be attached to a positional sensing device (60) comprises a handle (5) having at least one sensor (121, 122, 123) allowing a user of the device (10) to issue a handling signal of the data acquisition device (10). The handle (5) comprises at least two surface areas (110) adapted to be pushed in direction of the body of the handle (5). At least one force sensor (121, 122, 123) is associated to each of said surface areas (110) adapted to sense a pushing force of the user grasping and/or pressing the handle via the surface areas (110).

## Description

### TECHNICAL FIELD

The present invention relates to a data acquisition device representing a virtual object adapted to be attached to a positional sensing device according to the preamble of claim 1. Furthermore, the invention is related to a method and system for detecting and evaluating data relating to manipulation of a handle by a human subject.

### PRIOR ART

A stylus that has two switches that can be pressed by a subject manipulating the stylus to perform actions in a virtual environment, such as selecting virtual objects, is known from WO 2005/043365. Said stylus is a data acquisition device which can e.g. be attached to a positional sensing device which is preferably equipped with a feedback system. Such a positional sensing device is also disclosed in WO 2005/043365 and provides the functions of 6 DOF positional sensing and 3 DOF force feedback. The device according to the prior art can be used to evaluate the handling capability of subjects, especially from impaired patients. It is noted that this stylus provides the possibility of a user input through a switch and/or a push-button. The stylus can be replaced by a computer mouse or digitizer. The user interface according to WO 2005/043365 cannot measure the grasping force applied by the subject.

Daily activities of humans are strongly dependent on the use of arms and hands, and thus people suffering from functional deficits of the upper limb, e.g. following a stroke, are often severely impaired in the execution of simple tasks. The choice of appropriate therapy to recover lost abilities requires proper assessment of the functional deficits. However, known assessment tests of arm and hand function used in clinical routine (such as the Fugl-Meyer Motor Assessment Scale, the Box and Block test, the Jebsen Hand Function Test, etc.) suffer from important limitations, such as intra- and inter-rater variability, a limited amount or lack of quantitative measures, time-consuming administration, low responsiveness (ability to detect clinical changes over time) and low sensitivity (possibility for a large portion of patients to perform the test).

One known exercise to evaluate the possibility of a subject to execution of tasks using his arm and hand is the so called peg insertion test. Said test consists of moving nine pegs provided into a 3x3 array of holes having dimensions to accommodate the pegs, wherein the pegs are provided in a predefined starting position.

A peg board test is known from JP 2010/057629, provided for testing autonomous physical strength. The physical strength testing device has a peg board having a plurality of holes longitudinally provided in a row at both right and left ends and a plurality of holes longitudinally provided in two rows in the central portion and allowing a plurality of pegs to be inserted and pulled out of the holes. The peg board is put on a table and the subject of measurement inserts and pulls out the pegs between the holes in the right and left ends and the holes in the central portion. The time required in insertion and pulling out is measured. The measured time is compared with an evaluation criterion table previously obtained by experiments to test the subject's autonomous physical strength relating to the handiwork.

In "Abnormal joint torque pattern in the paretic upper limb of subjects with hemiparesis" by J.P.Dewwald and R.F. Beer, in Muscle Nerve, 24(2):273-283, Feb 2001, exoskeleton robotic devices have been used to investigate force generation or abnormal muscle synergies during reaching movements and to quantify abnormal shoulder and elbow coupling in stroke subjects.

"Movement smoothness changes during stroke recovery" by B. Rohrer et al. in J.Neurosci., 22(18):8297-8304, Sep 2002, investigates, while using planar robotic manipulanda, such as the MIT-Manus, movement smoothness in stroke subjects and found that movements tend to become smoother during recovery. Further attempts to investigate manipulation tasks and not only arm movements have been made using end-effector devices in which subjects were required to approach a virtual object in order to grasp it, as disclosed by P Feys et al. in "Arm training in multiple sclerosis using phantom: clinical relevance of robotic outcome measures" in Proc. IEEE International Conference on Rehabilitation Robotics, pages 576-581, 2009. However, these studies have some limitations such as low device transparency, limited degrees of freedom or do not fully represent functional tasks that require grasping, transport of an object and precise placement..

WO 2011/036350 discloses a device for evaluating and/or reinforcing gripping strength. Said device comprises electronic circuits built into a hollow space and consists of a functional combination of a pressure-measuring system with a wireless communication system and a power store for powering the pressure-measuring system and the wireless communication system. The self-contained object has a spherical shape, being made of a flexible airtight material, and having a size suitable for being grasped and compressed by a single hand. The airtight system has the advantage to react on any change of pressure by a hand grasping the device and the disadvantage that it can not be easily adapted to a haptic feedback system, in view of the hollow gas pressure based detection sensor, necessitating the flexible airtight outer sphere.

### SUMMARY OF THE INVENTION

Based on the prior art it is one object of the invention to provide an improved data acquisition device and an improved method of the peg test.

Said object is achieved through the features of claim 1.

Based on the prior art it is a further object of the invention to provide an improved method and system for detecting and evaluating data relating to manipulation of a handle by a human subject

Said object is achieved for a method through the features of claim 6 and for a system through the features of claim 9.

The Virtual Peg Insertion Test of the present invention allows assessing sensorimotor functions of arm and hand using an instrumented tool, virtual reality and haptic feedback. Such a method and device allows deriving a plurality of performance parameters from kinematic and kinetic data, which can be compared between two groups of healthy subjects performing the task with the dominant and non-dominant hand, as well as with a group of chronic stroke subjects suffering from different levels of upper limb impairment.

The invention combines virtual reality and an augmented haptic interface that can provide kinesthetic feedback through an instrumented handle grasped by the subject to provide a better tool for the assessment of arm and hand function. The test according to the system consists of a functionally relevant pick and place task. It combines reaching and grasping movements and thus involves both proximal and distal parts of the limb. During the task, the position and the orientation of the hand, the grasping force and the collision forces with the virtual board are measured in function of time. These measures can provide information about movement smoothness, joint coordination, muscle strength and sensory perception, all parameters found to be important in clinical assessments.

The present testing device and related method provides an objective, reliable and sensitive test for the assessment of arm and hand function. Said robotic device improves current clinical assessments since it provides a platform for objective measures of impairment. The system can precisely record movement trajectories, execution time and interaction forces during well-controlled and repeatable motor tasks to characterize movements of neurologically disabled subjects.

The device has been used to acquire grasping force profiles from tests with stroke subjects. The stored grasping force profiles show significantly smaller grasping forces applied by them compared to the healthy subjects and suggest a poor coordination between position and grasping for the stroke subjects. The collision forces with the virtual board were found to be indicative of sensory deficits. Since these results improve with training of the patients, the analyzed parameters are valid indicators of impairment and its development.

The aim of the invention is to improve assessment of hand and arm function of neurologically disabled patients using a haptic device in combination with an instrumented handle according to an embodiment and with a novel data analysis method. The patient's motion and forces are measured by the robotic device, which in turn will provide visual and haptic feedback to render more realistic conditions during virtual object manipulation.

The device allows for measurements with healthy and stroke subjects to evaluate the sensitivity of the test by comparing performances of healthy subjects executing the task with the dominant hand versus the non-dominant hand and to obtain baseline measures. Furthermore, these measurements aimed at determining the types of stroke subjects able to perform the task as well as identifying meaningful performance parameters representative of important sensorimotor functions.

The invention provides the device and method to execute a Virtual Peg Insertion Test, an objective, easy and fast to administer assessment test of arm and hand function using a haptic interface and an instrumented handle which can measure grasping forces. Measurements with several stroke subjects and found that subjects with some remaining fine motor skills could perform the Virtual Peg Insertion Test. Nine parameters related to task performance are compared within test results of stroke subjects and healthy subjects performing the task with the dominant and non-dominant hand. Eight of the nine parameters analyzed showed differences between the groups of healthy and stroke subjects and thus have the potential to be valid indicators of functional impairment. Previous studies have demonstrated how similar parameters could be indicative of some impairments, such as sensory deficits, muscle weakness and abnormal joint coordination, as discussed in more details below.

The data acquisition device represents a virtual object which can be represented for the user on a screen. It is at the same time a real object comprising a handle and is adapted to be attached to a positional sensing device. Said positional sensing device is optionally and preferably also a force feedback device. The handle incorporates at least one sensor, usually at least two or three sensors, allowing a user of the device to issue a handling signal when grasping the handle. The handle now comprises at least two surface areas adapted to be pushed in direction of the body or core of the handle. The mentioned at least one force sensor is associated with one surface area which can be thus adapted to sense a pushing force of the user grasping and/or pressing the handle via the surface areas. Preferably one force sensor is associated to one corresponding surface area, so that grasping forces from more than one direction can be sensed.

The handle can have the outer form of essentially a sphere, wherein the surface areas are then part-spherical surfaces, attached resiliently to the body of the handle. If there are two surface areas these are essentially semi-spherical surfaces separated by a slot. If there are three surface areas these are essentially part-spherical surfaces separated by slots. Preferably the three surface areas join at the "poles" of the handle and cover 120 degree along a half great circle.

The method for detecting and evaluating data relating to manipulation, by a human subject, uses a data acquisition device comprising a handle and a haptic interactive representation on a display of a computer unit. Then the position of a hand of a user is sensed in real space and force feedback to said user is provided using a haptic interface device. Signals are obtained through force sensors within the data acquisition device when said handle, attached to the haptic interface device, is grasped by the user. A graphical representation of a predefined number test objects to be displaced on the screen of the computer unit from one predefined place to another through real movement of the hand of the user grasping said data acquisition device is provided, wherein the progress of the test depends on the input provided through signals of the data acquisition device and the position and orientation signals provided by the haptic interface device. The haptic device delivers a force feedback action when the representation of the data acquisition device within the representation on the screen collides with other objects in the virtual space, based on control signals issued by the computer controlling the displacement of the test objects when the data acquisition device is grasped with a force over a predefined threshold detected by the force sensors. Said computer unit further gathers force data from the force sensors together with related time data and position data of the test objects to be used to evaluate the test progress.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematic view of a force feedback haptic interface comprising a data acquisition device according to an embodiment of the invention;
- Fig. 2: shows A) a section and B) a perspective view of a first embodiment of a two-portion handle according to the invention;
- Fig. 3: shows A) a section and B) a perspective view of a second embodiment of a three-portion handle according to the invention;
- Fig. 4: shows a schematic representation in a front view on a display of a typical movement of a virtual peg in the test according to the invention and typical parameters used to evaluate the results of the test;
- Fig. 5: shows trajectories of a representative healthy subject and a stroke subject during the execution of a complete trial of the Virtual Peg Insertion Test;
- Fig. 6: shows the grasping force in function of time of a healthy subject and a stroke subject (S1) during the execution of a complete trial of the Virtual Peg Insertion Test; and
- Fig. 7: shows a flowchart of the method of a virtual peg test according to the invention.

### DESCRIPTION OF PREFERRED EMBODIMENTS

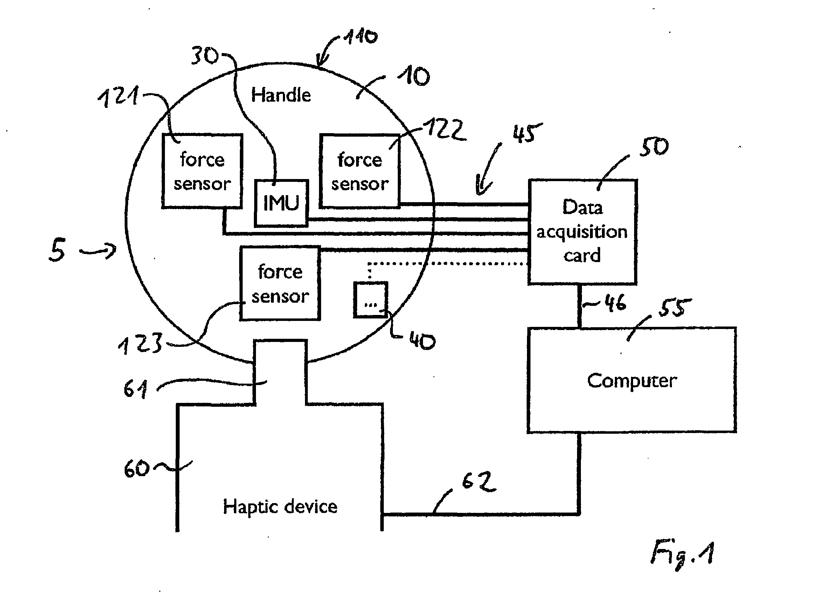

Fig. 1 provides a schematic view of a force feedback haptic interface comprising a data acquisition device 5 according to an embodiment of the invention; The data acquisition device 5 allows detecting data from users grasping said device via a handle 10. The handle 10 can e.g. be attached to a positional sensing device 60 via a docking station 61 providing the connection with the positional sensing device 60. The positional sensing device 60 is preferably equipped with a feedback system and constitutes a haptic device. Such a sensing device 60 is available from PHANTOM Omni, SensAble Technologies, Inc., USA and the function of its 6 DOF positional sensing and 3 DOF force feedback elements are described in WO 2005/043365. The data provided by that device are communicated via line 62 to a computer 55 and said line is also adapted to provide force feedback information to the haptic device 60.

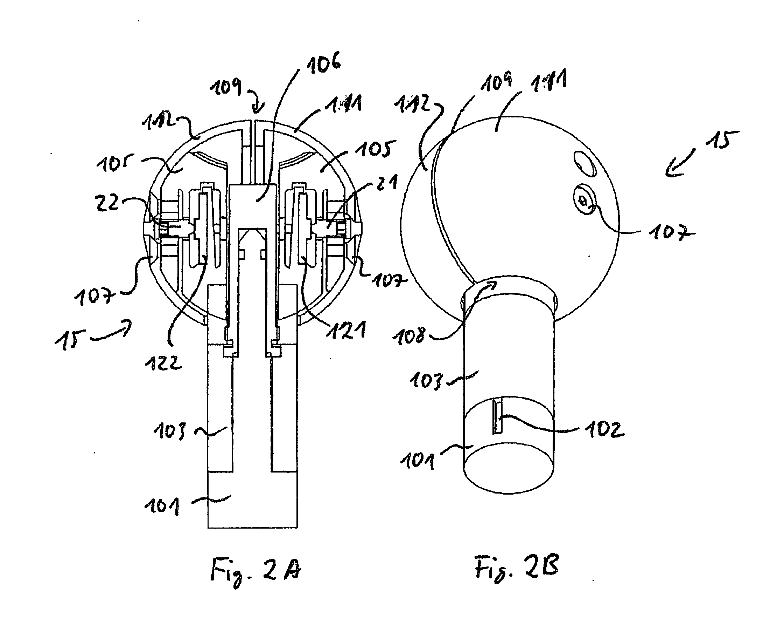

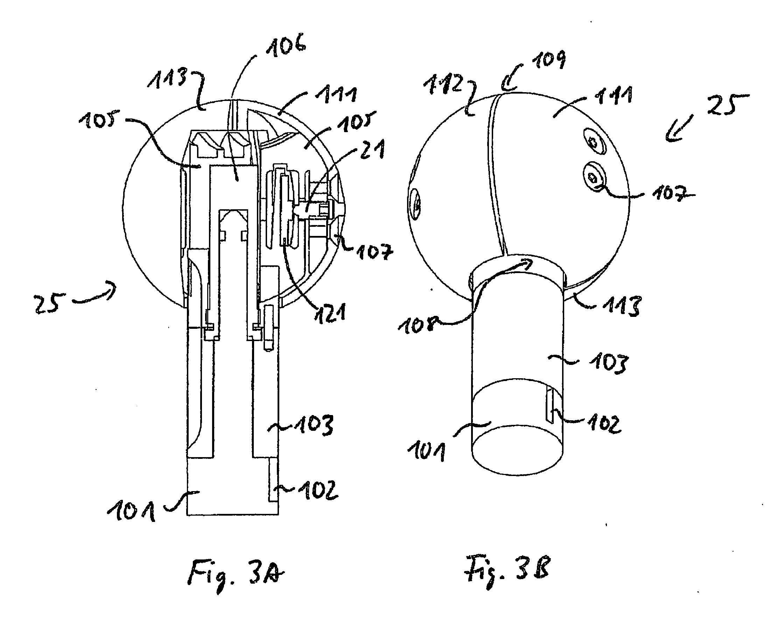

Fig. 1 provides a functional representation of the handle 10 including in an embodiment three single-axis force sensors 121, 122 and 123 (CentoNewton 40, EPFL, Switzerland) placed on a parallel flexible structure in order to measure the grasping force exerted on surrounding surface areas of the handle, which surface areas have the general reference numeral 110 in Fig. 1 and will be seen in more detail in connection with Fig 2 and 3. Said sensors are piezoresistive force sensors. They provided in an application a linear relationship between the applied force [Newton] and the output voltage [Volt] of one force sensor during repeated dynamic loading/unloading Voltage = 0.0915 * Force + 0.726 with a coefficient of determination R² = 0,9987.

Furthermore the data acquisition device 5 comprises an inertial measurement unit 30, or IMU, to report on the handle's velocity and/or acceleration, which can be realized using a combination of accelerometers. The optional IMU 30 comprises three accelerometers. The three accelerometers are placed such that their measuring axes are orthogonal to each other. Preferably their measuring axes are oriented in the same direction as the axes of the force sensors 121, 122 and 123. Finally additional measurement devices 40, which may comprise three gyroscopes, can be included in the handle 10.

All measurement devices 121, 122, 123, 30 and 40 are connected to the computer 55, usually via supply and data lines 45 and treated with a data acquisition card 50. Although the lines 45 show a separate connection via the data acquisition card 50 and data lines 56 to the computer 55, it is clear that these lines 45 are preferably integrated via the docking station 61 or a connection with the positional sensing device 60, or they can also be implemented with a wireless connection.

Fig. 2 shows A) a section and B) a perspective view of a first embodiment of a two-portion handle 15 according to the invention. The handle 15 comprises an adapter 101 being the core of the handle or data acquisition device 15. A groove 102 may be provided to attach adapter 101 to the docking station 61 of the haptic device 60, since the haptic device 60 provides the position detection and the force feedback function, relating to position and orientation (rotation) of the data acquisition device 15. The adapter 101 is surrounded with a mantle 103 providing a rod prolongation of the docking station 60. In this context it is only important that this adapter 102 provides the basis for the two semi-spherical handle portions 111 and 112, mounted on the core of the adapter 101. The portions 111 and 112 are separated one against the other by a slit 109 wherein they arc mounted with a round slit 108 at the basis of the adapter 101 and mantle 103.

The hollow semi-spherical handle portions 111 and 112 are attached with two screws 107 on a multi-slotted support 105. Each support 105 provides the connection between the hollow semi-spherical handle portion 111 or 112, respectively, and the core sleeve 106. Beside a flat structure mainly in the plan of the drawing of Fig. 2A it sis preferred that it covers a room angle up to the semi-sphere behind the cover handle portion 111 or 112, respectively.

In each support 105 one force sensor 121 or 122 is mounted, respectively, wherein a pretension screw 21 or 22 is provided within the support 105 against the sensor 21, 22. The support 105 is connected with the hollow central sleeve 106 mounted on the adapter 101. The slotted support 105 allow for the sensors 121 and 122 to be accommodated and to be actuated when a semi-spherical handle portions 111 or 112 is pushed against the central sleeve 106. The design of the present embodiment has a spherical shape which is easy to grasp and manipulate by stroke subjects with impaired hand function. The diameter of the sphere built by the semi-spherical handle portions 111 and 112 can be adapted to different hand sizes; usually it is chosen to have a diameter between 5 and 10 centimetres.

Fig. 3 shows A) a section and B) a perspective view of a second embodiment of a three-portion handle 25 according to the invention. The handle 25 comprises an adapter 101 being the core of the handle or data acquisition device 25. As explained with Fig. 2 the groove 102 attaches adapter 101 to the docking station 61 of the haptic device 60, not represented in the drawings of Fig. 2 and 3. Reference is made to the attachment in WO 2005/043365. Adapter 102 with its surrounding hollow cylindrical sleeve 106 provides the basis for the three part-spherical handle portions 111, 112 and 113 comprising approximately 120 degree of the circle. The three portions 111, 112 and 113 are joining at a single point opposite to adapter 101, separated one against the other by a slit 109 wherein they are mounted with a round slit 108 at the basis of the adapter 101 and mantle 103.

The hollow handle portions 111, 112 and 113 are attached with two screws 107 each on a multi-slotted support 105. Each support 105 attaches the corresponding hollow handle portions 111, 112 or 113 to the core 106, wherein the slots within the portions allow mounting of force sensor 121, 122 or 123, respectively, and compression of these sensors when the corresponding handle portions 111, 112 or 113 is pushed, generating a signal proportional to the mean force exerted on the corresponding handle portion. This design having three handle portions allows an improved signal generation, since three different signals oriented at an angle of 120 degrees in the plane of the longitudinal direction of the pretension screws (only screws 21 and 22 are shown in the drawings). Thus even small pressures of parts of the muscles of a subject grasping and manipulating the handle 25 are detected.

The devices 15 and 25 according to Fig. 2 and 3 were tested with a haptic device 60 according to WO 2005/043365, wherein it communicated with the computer 55 over a PCMCIA FireWire card. The grasping force is acquired through a USB data acquisition card 50. The data and supply lines 45 were provided not integrated with the docking station 61.

The data acquisition device 5 according to Fig. 1,, i.e. the handles 15 or 25 according to Fig. 2 or 3, respectively, attached to the positional sensing device 60, allows execution of a Virtual Peg Insertion Test consisting in grasping nine virtual pegs, one after the other, and inserting them into nine holes as quickly as possible. This test combines virtual reality and haptics. The haptic interface 60 provides kinesthetic feedback to the subject's hand in order to render a realistic reconstitution of the interaction with a real environment and precisely tracks the movement.

As mentioned above the sensors 121, 122 and 123 were dynamically loaded and unloaded (up to 100 N/s) to three force levels (approximately 10, 20 and 30 N) against a commercial load cell (Mini 40, ATI Industrial Automation, USA) while the voltage output of the piezoresistive sensor was measured. The measured force data were lowpass filtered at 50 Hz and show good linearity between the applied force [Newton] and the output voltage

[Volt].

Grasping forces are acquired over a USB data acquisition card (NI USB 6008, National Instruments, USA) while the position, angles and collision forces are acquired through a firewire connection, which also sends the commands from the physics engine for the forces to be displayed to the device. The task is implemented in Microsoft Visual C++ and OpenGL is used for graphic rendering. All signals are sampled at a rate of 1 kHz and are stored on a laptop (IntelCore 2, 2.67 GHz, Windows XP) as computer 55. The connection diagram is shown in Fig. 1. The complete setup is compact and can be easily transported and integrated in a clinical environment. The sensor data is stored within memory of the computer 55, especially during the test procedure and preferably from the group of force data (exerted by the hand of the user through grasping the handle), position data (of the handle), path data (of the handle in the real world or its representation of a cursor in the virtual world and of the pegs), rotation data (of the handle and/or pegs), velocity data (of the handle), acceleration data (of the handle), angular velocity data (of the handle) and angular acceleration data (of the handle), wherein preferably the data is always stored in connection with time data to reference the progress of the test.

The device was tested with three groups of subjects performing the Virtual Peg Insertion Test, two groups of 8 healthy subjects each and one group of 14 chronic stroke subjects. The first group (three females and five males, age 29±5 years) performed the task with their dominant hand, the second group (one female and seven males, age 29±3 years) with the non-dominant hand and the group of stroke subjects (three females and eleven males) with their impaired hand. Stroke subjects were recruited on a patient-by-patient basis from the ZAR in Zurich, Switzerland (Center for ambulatory rehabilitation, Zurich). The ZAR classifies patients into four categories based on their motor impairment level as described in Table I. Subjects of all four levels of impairment were tested (one A0, two A1, three A2 and eight A3). Five subjects reported sensory deficits in the hand during a pre-session interview. All subjects were required to have normal vision, 3D perception, the ability to understand the task and had to provide informed consent. A0 relates to a subject with "no arm movement"; A1 relates to "arm movements are significantly restricted"; A2 relates to "fine motor skills are restricted; impairment is pronounced"; and A3 relates to "fine motor skills are restricted; impairment is mildly pronounced".

Subjects were seated in front of a laptop and held the handle 15 or 25 attached to a device according to WO 2005/043365 in its initial position with the elbow flexed about 90 degrees and the shoulder abducted about 45 degrees. A blue board with a virtual board plane 200 was displayed on the screen of the laptop 55 with nine pegs 210 aligned vertically on the left which had to be moved and inserted into nine holes 220 displayed in a 3x3 matrix on the right.

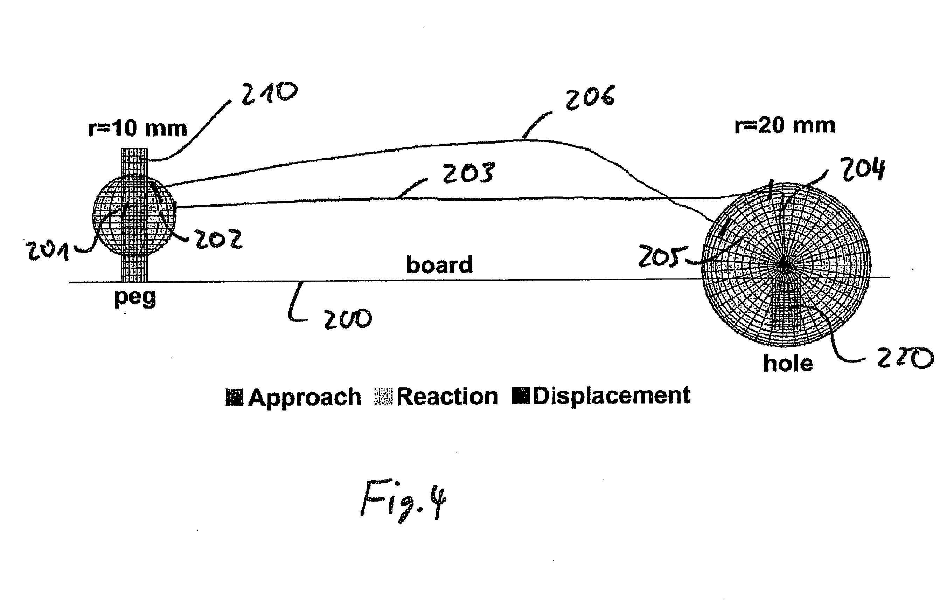

Fig. 4 shows a schematic representation of a display of a typical movement 201, 202, 203, 204, 205 and 206 of a virtual peg 210 to a virtual hole 220 in the test according to the invention and typical parameters used to evaluate the results of the test.

To execute the task, the subject had to manipulate the handle 15 or 25 which was represented by a cursor (not represented in Fig. 4) on the screen. The cursor had to be properly aligned with a peg 210 before grasping, and the (virtual) peg 210 would fall on the (virtual) board plane 200, if the grasping force was not maintained above a predefined threshold, e.g. 5 Newton.

The cursor has a specific color according to the progress of the experiment. Information about the different colors of the cursor and the pegs 210 was given to the subject before the experiment was started. The cursor is transparent yellow when no peg 210 is held, turns orange to indicate that it is properly aligned with a peg 210, green when a peg 210 is currently held and red when excessive grasping force is applied to the handle but no peg 210 is held. The subject was instructed to insert the nine pegs 210 into the nine holes 220 as fast and as precisely as possible using only the tested hand. In order to insert a peg 210 into a hole 220, the grasp had to be released below said force threshold. The test was completed once all pegs 210 were inserted into the nine holes 220. The pegs 210 could be taken in any order and inserted into any free hole 220. The difficulty of the task was adapted to the level of impairment of the stroke subjects. To do so, two task parameters were varied: the grasping force threshold, calculated as the mean of the three force sensors (between 1 and 5 Newton, wherein the value of 5 Newton was used for healthy subjects), and the maximal distance between the real position of the handle, represented by the virtual position of the cursor and the corresponding virtual peg position for the alignment (between 3 and 10 mm, wherein the value of 3 mm was used for healthy subjects). The alignment was calculated as the mean of the distance between the top of the cursor and peg 210 and the distance between the bottom of the cursor and peg 210 in order to account for both the position and the orientation.

During a recording session, subjects performed two test trials during which they received the instructions and were allowed to experience the force feedback. This was followed by five repetitions of the test (two for the strongly impaired stroke subjects).

During the execution of the Virtual Peg Insertion Test, the position profile of Fig. 4 shows a specific pattern with lines 201, 202, 203, 204, 205, 206 that is repeated for each peg 210. of course, the display is preferably 3D, so that the (virtual) pegs could be aligned in a line in the depth on the screen and the nine holes 220 are arranged in a 3x3 array on the 3D board plane 200. The subject displaces this handle 15 or 25 attached to the haptic device. This is translated by a proportional movement of the cursor on the screen of the computer 55. If due to a real movement of the handle 15/25 the cursor alone or the grasped peg 210 touches the board plane 200 or the hole or a/another peg 210 than the haptic device 60 provided a force feedback to the user, e.g. stopped the movement of the handle 15/25, if the cursor touched the board plane 200. In other word, the Virtual Peg Insertion Test uses a haptic interface 60 to render physical interactions with the environment (e.g. board 200, pegs 210 and holes 220) while it records kinetic and kinematic data.

The movement of transfer of a peg 210 into a hole 220 on the screen and return to fetch a new peg 210 can be decomposed into six sequences as follows: approach of the cursor to the peg for alignment and grasping 201, reaction phase once the peg is grasped 202, coarse displacement up to a hole 203, approach of a hole to insert the peg 204, reaction phase after insertion 205 and coarse displacement to the next peg 206, wherein the different phases are separated by short lines crossing the two adjacent sequences.

Fig. 4 shows such a front view of the decomposition of one trajectory into approach, reaction and displacement phases based on a spherical area 231 around the peg 210 with r=10 mm, centered on the middle of a peg 210 at its initial position and on a spherical area 232 around the hole 220 with r=20 mm, centered on the middle of a peg 210 when inserted in the hole 220. Of course different and especially smaller radii can be chosen.

From the movement data, nine parameters were calculated and compared between the different groups of subjects to evaluate arm and hand function. Some of the parameters were restricted to specific phases of the movement and averaged over the nine trajectories.
- The execution time Tₑₓ is the time to execute the task from the approach of the first peg 210 to the insertion of the last peg 210.
- The grasping force is calculated from the mean of the three force sensors integrated into the handle 15 or 25. The average grasping force F_{g} is calculated during the coarse displacement 203 and 206, giving one value for the transport of a peg 210 and another value for the return.
- The number of zero-crossings of the acceleration is normalized by the duration of the movement during the go and return displacement (N_{zc}).
- The number of times a peg 210 is dropped during the transport is counted (N_{dp}).
- The trajectory error Eₜᵣₐⱼ corresponds to the distance between the trajectory and the straight line in the horizontal plane normalized by the trajectory length during displacement (go/return).
- The mean collision force F_{cmean} represents the averaged force exerted against the board 200 during the task from the approach of the first peg 210 to the insertion of the last peg 210. The collision force is estimated from the motor currents required to render the haptic feedback during the collision with the board 200.

For each parameter, a one-way analysis of variance (ANOVA) was performed to test for statistically significant differences between the two groups of healthy subjects performing the task with the dominant versus non-dominant hand. The level of significance was set to 0.05.

The results of the analysis show a hand dominance in healthy subjects.

The performance parameters of the two groups of healthy subjects were first compared in order to determine if the performance significantly changed when the task was performed with the dominant versus the non-dominant hand. For each parameter, the mean values and the standard deviation for all subjects and all trials were calculated. The results of this analysis are presented in the following Table, showing performance parameters of subjects performing the task with the dominant versus the non-dominant hand and significant differences between the two groups (left). performance of four stroke subjects performing the task with the impaired hand (right) hand dominance is indicated as "d" for dominant and "nd" for non-dominant.

| | Healthy | | | Stroke | | | |
|---|---|---|---|---|---|---|---|
| Performance parameters | N=8 D | N=8 ND | p value | S1 ND | S2 D | S3 ND | S4 ND |
| Tₑₓ [s] | 25.8±6.6 | 26.6±7.1 | 0.66 | **93.0** | **48.5** | **76.0** | **149.1** |
| F*_{g}* [N] (go/return) | 15.0±5.1 | 12.5±3.5 | **0.0002** | **6.3** | 7.3 | 9.6 | **7.4** |
| | 0.73±0.42 | 0.80±0.58 | 0.32 | 0.83 | 0.33 | 0.99 | 1.02 |
| N*_{zc}* (go/return) | 7.5±1.9 | 6.9+1.5 | 0.22 | **16.4** | **17.4** | **14.0** | **8.5** |
| | 8..0±1.4 | S.6±1.7 | 0.24 | **14.5** | **12.9** | **13.9** | **13.9** |
| N*_{dp}* [peg/trial] | 0.13±0.28 | 0.05±0.09 | 0.40 | 0 | 0 | 0 | **0.5** |
| E*ₜᵣₐⱼ* [cm] (go/return) | 0.24±0.13 | 0.28±0.12 | 0.12 | **0.42** | 0.23 | 0.17 | **0.52** |
| | 0.50±0.14 | 0.43±0.18 | 0.08 | 0.42 | 0.43 | 0.31 | **0.78** |
| *Fₑₘₑₐₙ* [N] | 0.70±0.43 | 0.63±0.41 | 0.47 | 0.37 | 0.29 | **1.82** | **1.28** |

Small differences were found among the two groups. For example, the group performing the task with the non-dominant hand needed more time to complete the task, but this difference was not significant. Only the grasping force applied on the handle was found to be significantly smaller for the group performing the task with the non-dominant hand compared to the group performing the task with the dominant hand (p=0.0002). These results reveal that hand dominance is to be taken into account when analyzing the grasping force, but not for the other parameters.

Further results relating to test sensitivity in the stroke subjects were derived. Among the 14 tested stroke subjects, subjects with no remaining motor function in the arm (A0) or significantly restricted arm movements (A1) could not complete the task without assistance. However, A1 subjects could perform the task when an arm support compensated for the weight of their arm. Stroke subjects with remaining fine motor skills (A2 and A3) could perform the task with appropriate task parameters adjusted to their impairment level, meaning that the grasping force threshold and the alignment tolerance were adjusted. For valid comparison in the following analysis, only the four strokes subjects (S1-S4) who could perform the task with the same difficulty level as the two groups of healthy subjects (impairment level S1: A2, S2: A3, S3: A2, S4: A3) are used.

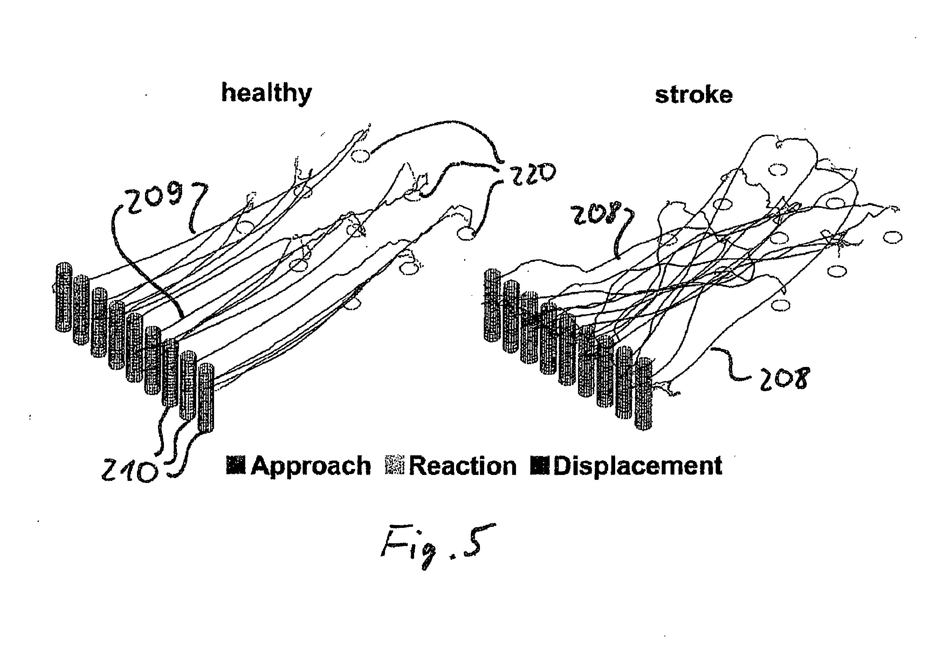

Fig. 5 shows the trajectories 208 and 209 of a representative healthy subject and a stroke subject (S1) during the execution of a complete trial of the Virtual Peg Insertion Test. The trajectories 208 or 209 are divided into the different task sequences: approach of a peg, reaction after taking a peg, coarse displacement during the transport of a peg, approach of a hole, reaction after inserting a peg in a hole and coarse displacement during the return according to Fig. 4.

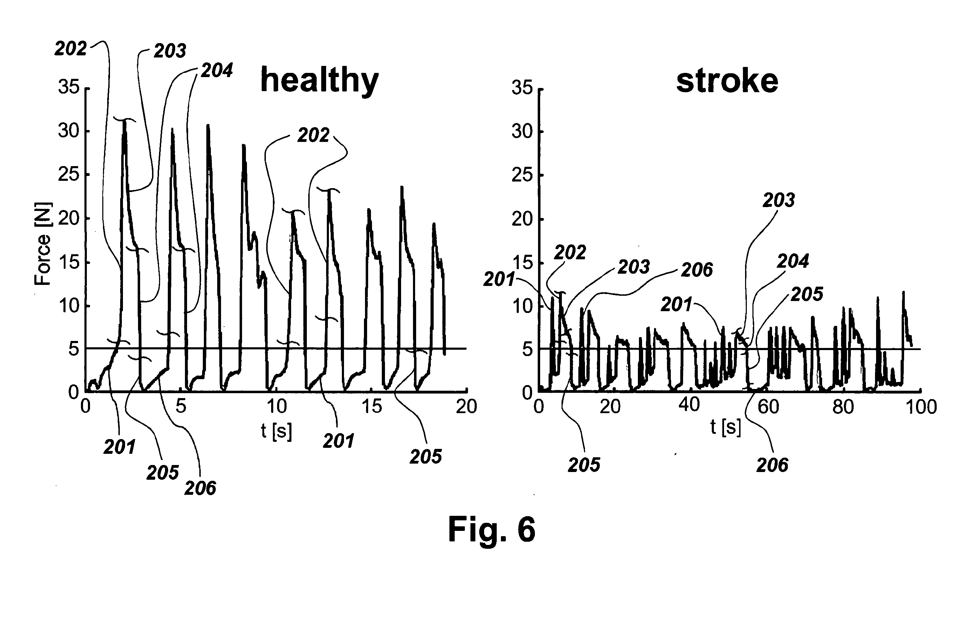

For a comparison between healthy and stroke subjects, performance of the stroke and the healthy subjects were compared in order to evaluate the parameters analyzed to be used as indicators of arm and hand impairment. Representative trajectories of a healthy subject and a stroke subject during one complete trial are presented. Healthy subjects tended to follow a straight trajectory 209 from the peg 210 to a hole 220 and little adjustments around the pegs 210 and the holes 220 were needed compared to stroke subjects as shown in trajectories 208. The grasping force in function of time is shown in Fig. 6 for the same trials.

Fig. 6 shows the grasping force in function of time of a healthy subject and a stroke subject (S1) during the execution of a complete trial of the Virtual Peg Insertion Test. The force threshold was set to 5 N as is indicated by the horizontal dotted line. Movement sequences are indicated as follows: approach 201 and 204, reaction 202 and 205, displacement 203 and 206 as explained in connection with Fig. 4.

The grasping force applied by the healthy subject on the handle clearly showed a repetitive pattern for the nine pegs 210. Healthy subjects tended to apply a strong force to initially grasp the peg 210 and slowly decreased the force during the transport of the peg 210. The mean grasping force applied by healthy subjects during the transport of a peg 210 was much larger than the threshold to hold the peg 210. The force profile of stroke subjects showed that they often needed several attempts to grasp the peg 210, represented by the peaks in Fig. 6. This was due to the application of force without proper alignment of the cursor with the peg 210. Additionally, the maximal forces applied on the handle by the stroke subject after taking a peg 210 were much smaller and the mean grasping force during the transport of a peg 210 was only slightly above the threshold of 5 N.

For each parameter, the mean values of all the trials were calculated for each stroke subject and are presented in the above represented table. For each parameter analyzed, the values differ from the healthy subjects for at least one stroke subject except for the grasping force during the return trajectory. The grasping force during the go trajectory tended to be lower for the stroke subjects while the execution time, the number of zero-crossings of the acceleration, the number of dropped pegs and the trajectory error tended to be higher. Two of the four stroke subjects (S3 and S4) suffered from sensory deficits and showed higher mean collision forces while the two other subjects showed normal values. Although some similarities can be seen, each stroke subject has his/her own set of impaired functions that has to be identified.

The execution time seems to vary with the severity of the impairment for the first three subjects with S2 (A3) being faster than S1 and S3 (A2). However, S4 (A3) was slower, which could indicate that hand sensory deficits also have an influence on the execution time. Grasping force could provide information on muscle strength and joint coordination. The grasping force profiles showed that stroke subjects applied less force during this kind of functional task, and the presence of several peaks during the approach of a peg indicated that they had difficulties in coordinating the position adjustment and control of force. Fine precision adjustments have been shown to be characteristic of stroke subjects. In the contrary, healthy subjects applied high forces, possibly with the aim of stabilizing their grip during peg transfer and maximizing their movement speed. Stroke subjects exhibited a higher number of zero-crossings of the acceleration, which has been shown to be a valid measure of movement smoothness and tends to decrease during recovery. Further, the trajectory error could correlate with an altered movement coordination and tends to become smaller with motor recovery. The results reveal that stroke subjects presenting sensory deficits showed higher collision forces with the virtual board 200 compared to the other subjects.

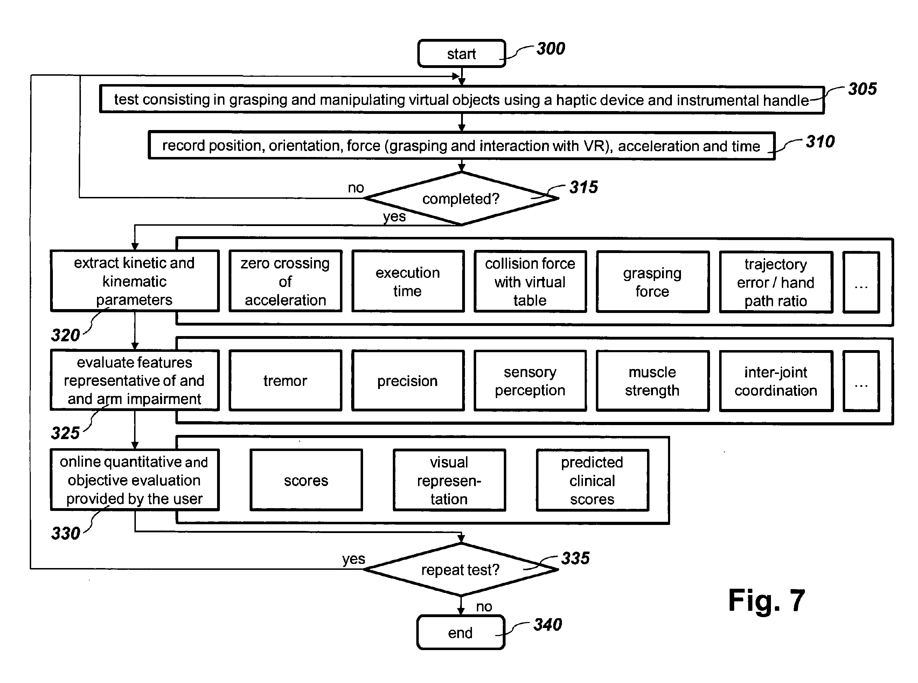

Fig. 7 shows a flowchart of the method of a virtual peg test according to the invention. After the start 300 of the test, the subject in question executes the displacement 305 of one peg 210 into one hole 220. The relevant data are recorded 310 in a first step, kinetic and kinematic data are collected with the neurologically impaired patient during well-controlled motor tasks involving hand, wrist and arm function (e.g. precise alignment grasping and manipulation of the pegs). This first step comprises the test 315, if the test is completed, i.e. all nine pegs 210 lodged in the corresponding holes 220.

In a second step, a set of motor performance indicators, for example the time required to complete a task, or the precision of a movement, will be extracted 320 from the kinetic and kinematic data. Said date can refer to zero crossing of acceleration, the execution time, the value of the collision force with the virtual board 200, the grasping force(s) and the trajectory error / hand path ratio, i.e. the deviation from a straight or directed movement.

In a third step, hand and arm impairment will be derived 325 from a model based on a combination of the performance parameters. It is essential to properly distinguish between impairment and performance since performance does not necessarily reflect the impairment, e.g. if patient uses compensatory strategies during the execution of a task. Impairment is defined here as the result of a disability, which has a direct consequence on what a patient can or cannot do with his/her arm and hand, while performance is defined here as how well one achieves a specific task. Such features can be calculated for tremor within the data, precision of the approach, the sensory perception, the related muscle strength and the inter-joint coordination.

Finally, an assessment scale 330 based on the subject's impairment is applied and can be compared to established clinical scales to help therapist select appropriate therapy for their patients, thus comprising scores, predicted clinical scores and the visual representation as in Fig. 5 and 6 for evaluation.

The use of a computer 55 supported procedure allow that all these steps are performed online giving the therapist and patient an immediate evaluation of arm and hand sensorimotor function immediately after the completion of the test.

### LIST OF REFERENCE SIGNS

- 5: data acquisition device
- 10: handle
- 15: two-portion handle
- 21,22: pretension screws
- 25: three-portion handle
- 30: IMU
- 40: further sensor
- 45, 46: supply and data lines
- 50: data acquisition card
- 55: computer
- 60: haptic device
- 61: docking station
- 62: line
- 101: adapter
- 102: groove
- 103: mantle
- 105: support
- 106: central sleeve
- 107: screw
- 108: round slit
- 109: slit
- 110: surface area
- 111,112,113: handle portion
- 121,122,123: force sensor
- 200: virtual board plane
- 201: approach peg
- 202: reaction on peg grasping
- 203: coarse displacement to hole
- 204: approach of hole
- 205: reaction on peg introduction
- 206: coarse displacement to peg
- 208: trajectory of a stroke subject
- 209: trajectory of a healthy subject
- 210: virtual peg
- 211: extent of computed
- 220: virtual hole
- 221: extent of computed
- 231: spherical area, peg
- 232: spherical area, hole
- 300: start of test
- 305: execution of a movement
- 310: recordal of data relating to movement
- 315: test of completed test
- 320: extraction of parameters
- 325: feature evaluation
- 330: score calculation
- 335: repetition of test
- 340: end of test

## Claims

1. A data acquisition device (10) representing a virtual object (210) and adapted to be attached to a positional sensing device (60), comprising a handle (5, 15, 25) having at least one sensor (121, 122, 123) allowing a user of the device (10) to issue a handling signal of the data acquisition device (10), **characterized in that**, the handle (5, 15, 25) comprises at least two surface areas (111, 112, 113) adapted to be pushed in direction of the body (101,106) of the handle (5, 15, 25), **in that** at least one force sensor (121, 122, 123) is associated to each of said surface areas (111, 112, 113) adapted to sense a pushing force of the user grasping and/or pressing the handle via the surface areas (111, 112, 113).

2. The data acquisition device (10) according to claim 1, wherein the handle (5, 15, 25) has an outer form of essentially a sphere, wherein the surface areas (111, 112, 113) are part-spherical surfaces, attached resiliently to the body (101, 106) of the handle (5, 15, 25).

3. The data acquisition device (10) according to claim 2, comprising two or three surface areas (111, 112, 113) being essentially semi-spherical surfaces or covering a 120 degree arc of the handle (5, 15, 25)

4. The data acquisition device (10) according to any one of claims 2 or 3, wherein the force sensors are piezoresistive force sensors (121, 122, 123) provided between the body (101, 106) and the surface areas (111, 112, 113) to detect radially oriented forces in relation to the sphere form of the handle (5, 15, 25).

5. The data acquisition device (10) according to any one of claims 1 to 4, further comprising accelerometers (30), preferably oriented for detection of accelerations along the same axes as a corresponding force sensor (121, 122, 123).

6. A method for detecting and evaluating data relating to manipulation, by a human subject, of a data acquisition device (10) comprising a handle (5, 15, 25), while using a haptic interactive representation (200, 210, 220) on a display of a computer unit (55) comprising the steps of:
- sensing a position of a hand of a user in real space and providing force feedback to said user using a haptic interface device (60);
- providing signals through force sensors (121, 122, 123) within the data acquisition device (10) when said handle (5, 15, 25), attached to the haptic interface device (60), is grasped by the user;
- providing a graphical representation of a predefined number test objects (210) to be displaced on the screen of the computer unit (55) from one predefined place to another through real movement of the hand of the user grasping said data acquisition device (10), depending on the input provided through signals of the data acquisition device (10) and the position and orientation signals provided by the haptic interface device (60)
- providing control signals to the haptic device (60) for a force feedback action when the representation of the data acquisition device (10) within the representation on the screen collides with other objects (200, 210, 220) in the virtual space;
- wherein the computer unit (55) comprises a control unit issuing control signals controlling the displacement of the test objects (210) when the data acquisition device (10) is grasped with a force over a predefined threshold detected by the force sensors (121, 122, 123);
- wherein the computer unit (55) further gathers force data from the force sensors (121, 122, 123) together with related time data and position data of the test objects (210) to be used to evaluate the test progress.

7. The method according to claim 6, wherein sensor data is stored within the test procedure, especially from the group of force data, position data, path data, rotation data, velocity data, acceleration data, angular velocity data and angular acceleration data preferably in connection with time data.

8. The method according to claim 6 or 7, wherein the predefined force threshold detected by the force sensors (121, 122, 123) is defined as a mean force value of the sensors.

9. A system for executing the method according to one of claims 6 to 8 for detecting and evaluating data relating to manipulation of a handle by a human subject, comprising a haptic interface device (60) for sensing a position of a hand of a user in real space and adapted to provide force feedback to said user; a data acquisition device (10), attached to the haptic interface device (60) and comprising the handle to be grasped by the user to provide signals; a computer unit (55) adapted to provide a haptic interactive representation (200, 210, 220) on a display, comprising a graphical representation of a predefined number test objects (210) to be displaced on said screen from one predefined place to another through real movement of the hand of the user grasping said data acquisition device (10) abd further adapted to control said representation of the test objects (210) on the screen depending on the input provided through signals of the data acquisition device (10) and the position and orientation signals provided by the haptic interface device (60) and providing control signals to the haptic device (60) for a force feedback action when the representation of the data acquisition device (10) within the representation on the screen collides with other objects in the virtual space, wherein the computer unit comprises a control unit issuing control signals controlling the displacement of the virtual test objects in the virtual world, when the data acquisition device (10) is grasped with a force over a predefined threshold detected by force sensors (121, 122, 123) incorporated within the data acquisition device (10), wherein the computer unit (55) is adapted to gather force data from the sensors together with time data and position data of the test objects (210) to be used to evaluate the test progress.
